Europäisches Patentamt

**European Patent Office**

Office européen des brevets

(19)

(11) Publication number: **0 227 593**
**A1**

(12) **EUROPEAN PATENT APPLICATION**

(21) Application number: **86810529.7**

(22) Date of filing: **19.11.86**

(51) Int. Cl.⁴: **A 61 K 45/06**
A 61 K 31/16, A 61 K 31/70
//(A61K31/70,31:16)

(30) Priority: **25.11.85 GB 8528983**
**13.06.86 GB 8614469**

(43) Date of publication of application:
**01.07.87 Bulletin 87/27**

(84) Designated Contracting States:
**BE CH DE FR GB IT LI LU NL SE**

(71) Applicant: **THE HOSPITAL FOR SICK CHILDREN**
**555 University Avenue**
**Toronto Ontario M5G 1X8 (CA)**

(72) Inventor: **Gelfand, Erwin W., Dr.**
**81 Ava Road**
**Toronto Ontario M6C 1V6 (CA)**

**Freedman, Melvin H., Dr.**
**Hospital for Sick Children 555 University Avenue**
**Toronto, Ontario M56 1X8 (CA)**

**Cohen, Amos, Dr.**
**640 Roseland Avenue, Apt. 510**
**Toronto Ontario M5N 1K9 (CA)**

(74) Representative: **Meyer, Hans Rudolf et al**
**Patentabteilung der CIBA-GEIGY AG Postfach**
**CH-4002 Basel (CH)**

(54) **Use of chelating agents.**

㊼ The invention relates to the use of iron chelating agents,
particularly. deferoxamine. in connection with the synergistic
treatment of cancer, especially for the treatment of leukemia,
with cytostatically effective preparations and for the prevention
or mitigation of side effects which are caused by cytostatics.

EP 0 227 593 A1

**0 227 593**

**Description**

Use of Chelating Agents

Field of the Invention

The present invention relates to the use of iron chelating agents, particularly deferoxamine, for the treatment of cancer, especially for the treatment of leukemia, and for the prevention or alleviation of side effects which can occur in connection with the treatment of tumour diseases by the use of cytostatically effective compounds.

Background of the Invention

The treatment of cancer diseases by the use of cytostatically effective chemotherapeutica is becoming more and more extensive, both as a treatment on its own or together with other types of treatment, such as the surgical removal of tumour tissues and/or radiotherapy. New active substances having an increased and/or more specific effectiveness along with better compatibility are being continuously tested and introduced. It is however known that, in spite of the inreased therapeutic range, most cytostatically effective compounds give rise to a series of more or less grave side effects which are common to virtually all these preparations. Apart from the various side effects which in part are specific to certain preparations, such as myocardiotoxic effects, the occurring side effects on cells having a high rate of division, such as those of bone marrow, the gastro-intestinal tract and hair, are particularly strongly marked and frequent. The last mentioned effects in the treatment with cytostatics very often cause nausea, vomiting, diarrhea and loss of hair.

It can be envisaged that side effects are prevented or at least mitigated by the administration of less than normal amounts of cytostatics. The side effects concerned are in particular nausea and vomiting, as well as loss of hair. It was now found that the use of iron chelating agents in the treatment of cancer, especially leukemia, in combination with cytostatic drugs leads to a synergistic effect and therefor permits lower dosages of cytostatic agents. In addition this combination causes increased sensitivity of the cancer cells for cytostatic drugs.

DFO is widely used therapeutically as a chelator of ferric iron in disorders of iron overload [1]. As previously demonstrated, this drug is a potent inhibitor of the DNA synthesis of human B and T lymphocytes in vitro [2]. Micromolar concentrations of DFO decreased intracellular levels of deoxyribonucleoside triphosphates and prevented cells from going beyond the early S-phase of the cell proliferation cycle [2]. We also found that DFO could irrirversibly inhibit proliferation and colony formation in the leukemia cell line HL-60 and induce differentiation and DNA damage in these cells [3].

These data encourage to test the potential use of DFO in leukemia chemotherapy, either as a single agent or in combination with other cytotoxic drugs. We wish to report the first therapeutic application of DFO in the treatment of leukemia in a 6 week old patient with acute leukemia. Studies performed on the patient's peripheral blood (PB) and bone marrow (BM) confirmed the antiproliferative properties of DFO in vitro and in vivo, as well as modulatory effects on leukemic cell differentiation.

Detailed Description

The present invention relates therefore to the use of iron chelating agents in combination with cytostatically effective preparations for the treatment of cancer, and concerns a therapeutical method for the treatment of cancer, characterized in that an iron chelator and a cytostatic agent in a cytostatically effective amount are consecutively or simultaneously administered to a patient suffering from cancer.

The present invention relates especially to the use of iron chelating agents in combination with cytostatically effective preparations for the prevention or mitigation of toxic side effects which are caused by cytostatics.

By iron chelating agents are meant in the first place compounds which have lowering effects on the content of freely available iron in body fluids and cells. Iron chelators are for example iron(III)-chelators, such as pyridoxal isonicotinoyl hydrazone (PIH; P. et al., Biochim. Biophys. Acta (1984) 802:477-489; Ponka, P. et al., FEBS Lett. (1979) 97: 317-321; Ponka. P. et al., Biochim. Biophys. Acta (1979 586:278-297),
diethylenetriaminepentaacetic acid (DTPA; Modell, B. and Berdoukas, V.. The Clinical Approach to Thalassaemia, p. 235-240, Grune and Stratton, London 1984).
phenolic ethylenediamine derivatives, e.g. N.N'-ethylene-bis(o-hydroxyphenylglycine (EHPG); N,N'-bis(o-hydroxybenzyl)-ethylenediamine diacetic acid (HBED). dimethyl-EHPG or dimethyl-HBED (Hershko, C. et al., J.Lab. Clin. Medicine (1984) 103: 337-346),
alpha-ketohydroxy heteroaromatic compounds, e.g. 1.2-dimethyl-3-hydroxy-pyrid-4-one and 2-methyl-3-hydroxy pyr-4-one and 2-hydroxypyridine-N-oxide (e.g.. 2.4-dihydroxypyridine-N-oxide) derivatives (Kontoghiorghes. G.J.. Lancet (1985) 2:817).
substituted polycyclotols. e.g. all-cis-1.3.5-triamine-2.4.6-cyclohexanetriol and its derivatives (Erni. I.et al.. Abstract. European Iron Club Meeting. Amersfoort. The Netherlands. Sept. 18-20. 1985),
desferri-ferrithiocin and derivatives thereof (EP 45 281) and. in particular, deferoxamine and derivatives thereof [Bickel et al., Helv. Chim. Acta 46. 1385-1389 (1963)]. preferably the methanesulfonate salt of deferoxamine. commercially available under the trade name Desferal®. Also other pharmaceutically acceptable acid addition salts can be used. e.g. the hydrochlorides. Further iron chelators are catecholamide iron chelators. e.g.

2

agrobactin, vibriobactin or parabactin and derivatives thereof. (Raymond J. Bergeron et al, Biochem. Biophys. Res. Comm., Vol. 121, 848-854 (1984); R. J. Bergeron et al., J. Org. Chem. 1983, 48, 3432-3439, S.R. Meshnik, et al., Biochem. Pharmacol. 34(17) 3147-3152, 1985).

The iron chelators used according to the invention can be applied in the form of pharmaceutical preparations able to be administered enterally or parenterally (intravenously, intramuscularily, subentaneously, e.g. by bollus injection or by infusion). The doses administered are those customarily used to obtain iron binding effects, for example from about 10 to 500 mg/kg/day, preferably about 200 to 300 mg/kg/day, preferably in form of intravenous infusions, whereby the dose can vary depending on the form of administration and the age, weight and general condition of the patient.

It has been shown that a lower amount of cytostatic is already effective if the iron chelator is administered, in particular if it is administered before and during the treatment with one or more of the cytostatically effective preparations. The iron chelator is usually administered before the treatment with the cytostatic starts, whereby the parenteral, especially intravenous, administration during about one to seven days before the treatment with the cytostatic(s) starts is preferred.

In connection with the present invention, it is possible to administer a wide variety of cytostatically effective preparations. The cytostatics are administered in the dosages and the intervals recommended for the appropriate cancer treatment, or in lower dosages, optionally also in combination. Reference is made in this connection to Brunner and Nagel, Internistische Krebstherapie (second edition, 1979, Springer Verlag. Berlin, Heidelberg, New York) and Gilman, A., et al. (eds), Goodman and Gilman's "The Pharmacological Basis of Therapeutics", Macmillan 10, New York (e.g. 7th Ed., 1985, p. 1249-1313).

The synergistic effect of the present combination advantageously permits the application of lower dosages than recommended up to now.

Cytotatics, applicable in connection with the iron chelators according to the invention are, inter alia, alkylating substances, such as mechlorethamine [nitrogen mustard or N,N-bis-(2-chloroethyl)-N-methyl-amine], triethylenephosphoramide [tri-(1-aziridinyl)-phosphine oxide], cyclophosphamide [2-[bis-(2-chloroethyl)-amino]-3,4,5,6-tetrahydro-2H-1,3,2-oxazophosphorine-2-oxide], ifosfamide [2-(2-chloroethyl)-amino-3-(2-chloroethyl)-3,4,5,6-tetrahydro-2H-1,3,2-oxazophosphorine-2-oxide], chlorambucil [4-{4-[di-(2-chloroethyl)-amino]-phenyl}-butyric acid], busulfan [1,4-di-(methanesulfonyloxy)-butane], melphalan [p-di-(2-chloroethyl)-amino-2-phenylalanine] or triaziquone [2,3,5-tri-(1-(1-aziridinyl)-p-benzoquinone], also nitrosourea compounds, such as carmustine [N,N'-di-(2-chloroethyl)-N-nitrosourea], or lomustine (CCNU; N-(2-chloroethyl)-N'-cyclohexyl-N-nitrosourea) or semustine (methyl CCNU). Also used are antimetabolites, such as methotrexate (amethopterine or L-(+)-N-[4-N-(2,4-diamino-6-pteridinyl)-methyl-N-methyl-amino]-benzoyl-L-glutamic acid), 10-Ethyl-10-deaza-aminopterin(10-EDAM), [N-[4-[1-(2,4-Diamino-6-pteridinyl)but-2-yl]-benzoyl]-L-glutamic acid], mercaptopurine [6-mercapto-7H-purine], thioguanine [2-amino-6-mercapto-7H-purine], cytarabine [Ara-C, cytosine-arabinoside or 1- β-D-arabinofuranosyl-cytosine], fluorouracil [5-fluoro-1H, 3H-pyrimidine-2,4-dione], floxuridine [5-fluorodeoxyuridine or 1-(2-deoxy-β-D-ribofuranosyl)-5-fluoruracil], or ftorafur [1-(2-tetrahydrofuryl)-5-fluoruracil]. A further group of cytostatics includes vinblastine [vincaleukoblastine] and vincristine [leucocristine], as well as certain antibiotics, such as actinomycin-D, daunorubicin (=daunomycin), doxorubicin [adriamycin], mithramycin [aureolic acid], streptonigrin, mitomycin and bleomycin. Further suitable cytostatics are, inter alia, procarbacine [4-(2-methyl-hydrazino)-methyl-benzoic acid-isopropylamide], hydroxyurea, [N-hydroxy-urea], L-asparaginase, dacarbazine (DTIC or 5-(3,3-dimethyl-1-triazenyl)-1H-imidazole-4-caboxamide], mitotane [o,p'-DDD or 2,2-dichloro--1-(4-chlorophenyl)-1-(2-chlorophenyl)-ethane], estramustine [3-0-(N,N-di-(2-chloroethyl)carbamoyl)-ostradiol], or podophyllotoxin.

Further cytostatic agents are hormones and hormone antagonists, such as corticosteriods, e.g. prednisone, progestins, e.g. hydroxyprogesterone or medroprogesterone, estrogens, e.g. diethylstilbestrol, antiestrogens, e.g. tamoxifen, androgens, e.g. testosterone, and aromatase inhibitors, e.g. aminogluthetimide.

The above-mentioned cytostatically effective compounds having salt-forming properties can optionally be used also in the form of their pharmazeutically applicable salts.

The cytostatically acting compounds are used in the form of pharmaceutically applicable preparations for oral or parenteral, such as intravenous, administration or in a form suitable for infusion. Preferably the iron chelator is administered before and eventually during administration of the cytostatic either continuously or optionally with interruptions. It is however also possible to use mixtures of the two agents.

In particular the ironchelator is administered during the first one to six or seven days, in dosages normally given to bind the freely available iron, and the cytostatic agent is administered starting at the third day. Preferably the cytostatic agent is administered in dosages lower than normally applied.

The invention is further defined in the accompanying claims.

The invention is based on the following more detailed in vitro and in vivo observations and experiments.

The following abbreviations are used:

DFO deferoxamine

DNA desoxyribonucleic acid

PB peripheral blood

BM bone marrow

Hb hemoglobin

WBC white blood cells

PAS periodic-acid-Schiff
ALL acute lymphoblastic leukemia
CFU-GEMM colony forming unit-granulocyte, erythroid, monocyte, magrophage
RPMI Roswell Park Memorial Institute
PHA-LCM phytohemoglutinin condition medium
BFU-E burst forming unit-erythroid
CFU-C colony forming unit-granulocyte
SRBC sheep red blood cell
AET aminoethylisothiouronium bromide
FCS fetal calf serum
PBS phosphate buffered saline
PHA phytohemaglutinin
HLA-D histocompatibility locus D
CALLA common ALLA
ANLL acute non-lymphocytic leukemia
Ph[1] Philadelphia cromosome
CFU-E colony forming unit-erythroid

Short Description of the Observations

A six week infant of 3.0 kg weight with congenital acute leukemia failed to attain remission with chemotherapy. Because we previously demonstrated that the iron chelator deferoxamine (DFO) has antiproliferative properties as well as modulatory effects on cell differentiation, a protocol was designed for in vitro study and for clinical use in the patient. At diagnosis, blast cells were morphologically undifferentiated, had non-diagnostic cytochemistry, showed an abnormal karyotype (t[4;11]), expressed markers of B-cell lineage, and demonstrated C$\mu$ gene rearrangement. Tissue cultures of marrow or blood cells yielded colonies of leukemic blasts. In vitro, increasing concentrations of DFO produced a dose-dependent suppression of patient blast colony growth. Blasts studied immunocytochemically from colonies exposed to DFO showed a marked change in surface antigen expression from lymphoid to myelomonocytic markers, became monocytic in appearance, and developed intense staining for non-specific esterase. When DFO was given intravenously to the patient as a single agent for 48 hours, blasts no longer expressed lymphoid antigens and became strongly positive for myelomonocytic markers, identical to the in vitro findings. Intravenous DFO halted rising peripheral blood blast cell numbers and allowed a several-fold increase in normal hematopoietic progenitor colony growth. When followed by low-dose (5 mg/kg/day) cytosine arabinoside in the treatment protocol, there was striking leukemic cytoreduction.

Our findings indicate that DFO in combination with cytostatically effective compounds increases in a synergistic manner their anti-leukemic properties by virtue of its effects on proliferation and differentiation.

Case Report

A six-week-old female infant of 3 kg weight was assessed for a paroxysmal harsh cough and was found to have a marked leukocytosis. She was the product of normal full-term pregnancy and the neonatal course was unremarkable. On physical examination she appeared well. The chest was clear to auscultation. Spleen tip and liver edge were palpable. There was no lymphadenopathy.

Her blood counts were as follows: Hemoglobin (Hb) 13.3 g/dL, white blood cells (WBC) 94,500/mm$^3$ with blasts comprising 52,400/mm$^3$, platelets 295,000/mm$^3$. Values for urea, creatinine, electrolytes, and uric acid levels were normal. Serum proteins, bilirubin, alkaline phosphatase and glutamic oxaloacetic transaminase levels were also normal. Cerebrospinal fluid was acellular with normal chemistry.

A bone marrow aspirate confirmed leukemic replacement. Blast cells were morphologically undifferentiated. Cytochemistry testing was negative for PAS, Sudan-black, non-specific esterase, and acid phosphatase. Immunologic marker studies on the diagnostic marrow cells were suggestive of B-cell origin: Ia, 80 %; B-4, 68 %; BA-1, 14 %; BA-2, 50 %; B-1, negative; CALLA, negative; cIgM. negative; sIg, negative, Myelo-monocytic markers (MO-1, My 906. My-4), T-cell markers (Leu-1) and E-rosette formation were all negative. Further evidence that the blast cells were derived from B-cell precursors was based on studies of immunoglobulin gene rearrangement. Following restriction enzyme digestion, a C$\mu$ probe recognized a rearrangement of one $\mu$ chain allete [4]. Cytogenetic analysis of 26 metaphases from unstimulated marrow cultured for 24 hours revealed a normal female karyotype (46XX in 4 cells) and a clone bearing a translocation involving the long arms of chromosomes 4 and 11 (in 22 cells). The breakpoint in chromosome 4 was in the region q21 and in chromosome 11 in the region q23.

A diagnosis of congenital acute lymphoblastic leukemia (ALL) was made and a four-week chemotherapy treatment protocol was started: vincristine i.v. weekly 4 x 0.21 mg; daunomycin i.v. weekly 3 x 3.5 mg; L-asparaginase i.m. 3 x weekly for 9 doses of 1680 units and cytosine-arabinoside i.t. 1 x 7.5 mg. At the conclusion of therapy, peripheral blasts were still present (42,200/mm$^3$) and a repeat marrow aspirate confirmed failure to attain remission. Peripheral blood and bone marrow blasts expressed identical immunological markers to those demonstrated on cells from the original marrow.

With informed consent and following an approval by the Human Experimentation Committee of our institution, an experimental protocol was initiated: DFO, dissolved in sterile water was given by continuous infusion of 10 mg/kg/hour; cytosine arabinoside (Ara-C) dissolved in saline was given by i.v. push, 5 mg/kg/dose. When used consecutively, DFO was stopped 8-12 hours prior to cytosine arabinoside. The protocol schedule and results of white blood cell and blast counts are provided in Table 1.

Materials and Methods for in vitro Tests

Deferoxamine: (desferioxamine methanesulfonate, Desferal® CIBA-GEIGY, Mississauga, ON) was dissolved in iron-free RPMI 1640 at a concentration of 10 mg/ml and added to cell cultures in final concentrations of 0.75 to 2.15 $\mu$ M.

Preparation of Cells: Heparinized BM or PB cells were layered over Ficoll-Paque (Pharmacia Fine Chemicals, Piscataway, NJ) and centrifuged (200 x g, 4°C) for 20 min. to eliminate neutrophils and red cells.

CFU-GEMM Assay: The CFU-GEMM assay was performed according to Fauser and Messner [5,6] with slight modifications. Briefly, 2 x 10⁵ nucleated PB cells were cultured in methylcellulose with iron-free RPMI (Ontario Cancer Institute, Toronto, Ont.), 30 % AB serum, 5 % leukocyte conditioned medium prepared with phytohemagglutinin (PHA-LCM) [7] with RPMI, human erythropoietin 1.0 U/ml (British Columbia Cancer Research Institute, Vancouver, BC) and 5 x 10⁵ M 2-mercaptoethanol. One ml of the culture mixture was placed in 35 mm Petri dishes and incubated at 37°C with 5 % $CO_2$ in air, in a humidified atmosphere. All cultures were evaluated after 14 days for the number of BFU-E colonies, defined as an aggregate of more than 500 hemoglobinized cells or 3 or more erythroid subcolonies, CFU-C colonies of at least 40 granulocytic or macrophage cells or both, and mixed colonies containing all elements. Individual colonies were plucked from the cultures with a micropipette and analyzed for cellular composition and immunological markers.

ALL Blast Colony Assay: The assay we used is a modification of previously described methods [8,9].
T-cell depletion: Post-Ficoll mononuclear BM cells were incubated with neuraminidase sensitized sheep erthrocytes (SRBC) at 4°C for 2 hours, followed by gradient centrifugation. This procedure was repeated twice. Cells at the interface contained less than 0.5 % T-cells as assessed by AET sensitized SRBC rosette formation [10] and using anti-Leu-4 and anti-Leu-5 monoclonal anti-T-cell antibodies (Becton Dickinson, CA). T-lymphocytes were obtained by recovery of rosetted cells from the pellet following lysis with Tris-buffered ammonium chloride (Sigma, St. Louis, MO).
Feeder Cells: Post-Ficoll normal donors' PB cells were depleted of adherent cells by incubation on plastic Petri dishes with 10 % fetal calf serum for 90 min. at 37°C. In this modification of the technique described by Shaw et al [11], non-adherent cells were removed by gentle pipetting and repeated vigorous washing of the plates with RPMI. This procedure was repeated until no further cells adhered to the plastic Petri dishes. The non-adherent cell fraction contained < 3 % monocytes according to analysis of cytocentrifuge smears counting 300-500 cells using Wright's stain, non-specific (alpha naphthyl butyrate) esterase stain [12], and a modification of an immunocytochemical technique [13-15] with anti-MY-4 monoclonal antibody (Coulter Immunology, Hialeah, FL).

Non adherent cells were incubated for 2 hours at 37°C, 5 % $CO_2$ in a humidified atmosphere. The cells were then quickly cooled to 6°C and exposed to 7000 rads irradiation using the Gamma cell 100 Blood Irradiator (Isomedix Inc. Parasippany, NJ).

Preparation of Phytohemaglutinin condition medium (PHA-LCM): Leukocyte-rich plasma from patients with hemochromatosis undergoing therapeutic phlebotomies was used. Post-Ficoll mononuclear cells were incubated with SRBC in 10 % FCS (heat inactivated at 56°C for 1 hr) at 37°C for 30 min. After incubation, a T-cell depletion was performed and the E+ layer was washed with PBS. SRBC were lysed with Tris-buffered ammonium chloride and rewashed. E+ cells were cultured in 250 ml tissue culture Falcon flasks (Becton-Dickinson, Oxnard, CA) with 1 % PHA (Wellcome HAIS, Dratford, England) at 37°C, 5 % $CO_2$ in a humidified atmosphere. Supernatant was collected after 72 hours of incubation, filtered and stored at 4°C [8].

Cell Culture Procedure: 2 x 10⁵ T-depleted cells were incubated with 40 % RPMI-methylcellulose, 20 % PHA-LCM, 10 % FCS, irradiated feeder cells (10⁵/dish), 16.6 % RPMI in 35 mm Petri dishes. The culture dishes were placed into a specialized modular incubator chamber (Billups-Rosenberg, Del Mar, CA) in a humidified atmosphere of 5 % $O_2$, 5 % $CO_2$ and balanced nitrogen for 3-6 days. After 2 days of incubation, the culture dishes were carefully examined. Colonies were counted at day 4. A cluster of more than 20 cells was defined as a colony. Individual colonies were plucked from the culture plates and smeared on microscope slides for further evaluation, using Wright stain and non-specific esterase staining. For control, 2 x 10⁵ irradiated feeder cells were cultured in the same assay system in each experiment to exclude their potential for colony formation

Immunocytochemical Method: The cell surface antigens of air dried smears were detected using monoclonal antibodies and intestinal alkaline phosphatase, as previously described [13-15]. This assay was performed to detect surface antigens on patients' PB and BM smears and colonies plucked from cultures. The slides were counter- stained in hematoxylin for light microscopy and the presence of surface antigen was indicated as bright red granular cellular inclusions. Morphologic details were well preserved, and a very high degree of correlation between morphology and surface antigen expression was observed. The intensity of staining was defined as strong (+ + +), moderate (+ +) and weak (+).
Immunofluorescence Method: The cell surface phenotype was determined by indirect cell-surface fluorescence: A fluorescein-conjugated goat anti-mouse IgG (FAb')₂ was used as second antibody. The labelling was performed at room temperature in the presence of 0.2 % sodium azide. The percentage of cells with surface fluorescence was determined by counting cells stained in three replicate wells using an inverted phase-fluorescent microscope.

Monoclonal Antibodies: The following monoclonal antibodies were used in these studies: anti-OKIa (Ortho Diagnostic Systems Inc. Raritan, NJ) to detect HLA-D framework antigen, anti-MY-7. anti-Mol and anti-MY-4 (coulter Immunology) to identify myelomonocytic markers, anti-Ig. anti-BA1, anti-BA2, anti-B1, and anti-B4 (Becton-Dickinson) to recognize cells of the B-cell lineage, anti-J5 (Coulter Immunology) to recognize cells bearing common ALL (CALLA) antigen and anti-Leu-1 and anti-Leu-4 (Coulter Immunology) to recognize cells of the T-cell lineage.

RESULTS

IN VITRO

Blast colony assay: Prior to therapy with DFO, patient's PB blast cells were cultured using the ALL blast colony assay.

The PB yielded a mean of 700 blast colonies/$2 \times 10^5$ cells plated. Individual colonies were plucked from the culture plates and were examined for cellular composition. Morphologically, they were undifferentiated blast cells identical to the appearance of marrow cells at diagnosis. The cells were negative for non-specific esterase by cytochemistry. Surface antigen studies of marrow cells confirmed lymphoblastic markers and absent myelo-monocytic markers (Table 2).

Anitproliferative Effect of DFO: As shown in Figure 1, when increasing concentrations of DFO were added to cultures of patient's PB blast cells, there was a dose-dependent suppression of blast colony growth. Significant decline in blast colonies was observed with DFO concentrations greater than 1.25 $\mu$M, and with 2.15 $\mu$M DFO, a 70 % reduction in blast colonies was observed.

DFO and Blast Cell Differentiation: Individual blast colonies from the culture plates containing DFO 2.5 $\mu$M were characterized (Table 2). Compared to colonies grown without exposure to DFO, a marked change in cell surface antigen expression was observed. Blast colonies from the DFO plates strongly expressed the myelo-monocytic markers OKM1 and MY-4 as determined by immunocytochemistry, and numbers of B-4 positive cells were markedly reduced. Moreover, individual cells from colonies exposed to DFO changed in morphology appearing monocytic, and staining intensely for non-specific esterase which could be inhibited by fluoride.

IN VIVO

DFO was administered therapeutically according to the protocol outlined in Table 1.

DFO and Blast Cell Differentiation: After 48 hours of continuous infusion of DFO as a single agent, striking changes in blast cell surface markers were detected immunocytochemically (Table 3). Prior to treatment, no blasts expressed OKM1 antigen, and only 8 % weakly expressed MY-4. After 48 hours, these two antigens were strongly detected in peripheral blood blast cells. Concomitantly with DFO therapy, there was a sharp decline from 37 % to 0 % of cells bearing $B_4$ antigen. These data were confirmed by immunoflorescence studies.

At the conclusion of the 20-day treatment protocol, a marrow aspirate showed 60 % blasts which were negative for PAS, Sudan-black, and acid phosphatase. In contrast to the marrow aspirate prior to DFO therapy, 60 % of the blasts were positive for non-specific esterase and staining could be inhibited by fluoride. Surface marker studies performed concurrently demonstrated expression of myelo-monocytic antigens which were not detected in marrow cells prior to treatment (Table 4).

Antiproliferative Effect of DFO: The WBC and blast number had increased several-fold in the three days prior to DFO treatment (Table 1). After the first 48 hours of DFO therapy. the counts reached a plateau. Significant decline of WBC and blast numbers was observed by 5 days following combination DFO-ARA-C therapy. By day 15, PB blasts almost completely disappeared.

Several CFU-GEMM assays were performed on PB during the first 48 hours of DFO (Table 5). Impressive increases in normal hematopoietic progenitor colony growth were documented. Erythroid and granulocytic progenitor colony formation increased several-fold over pre-DFO treatment colony numbers.

DISCUSSION

The infant who was studied had congenital leukemia that was unresponsive to conventional chemotherapy. At the conclusion of induction therapy, blasts were still present in PB and in marrow, and when chemotherapy was stopped, PB blast counts increased several-fold in three days.

The blasts were undifferentiated according to morphological and cytochemical criteria, and expressed antigens of B-lineage at diagnosis. Further confirmation of their origin from B-cells was their analysis of DNA from the leukemic cells which revealed rearrangement of one of the immunoglobulin heavy chain genes. The presence of an abnormal karyotype, t(4;11). as found on cytogenetic analysis of BM cells, has been previously reported on congenital ALL [16]. This form of leukemia is characterized by PB leukocytosis, blast cells with an undifferentiated appearance, and poor prognosis[17]. Although the cytogenetic abnormality has been found in ALL [18]. it has also been seen in cases of acute leukemia of myelo-monocytic lineage [14].

We were prompted to use DFO in an experimental treatment protocol because of our previous finding that the agent was a reversible early S-phase inhibitor of human lymphocyte proliferation [2]. Although widely used as a chelator of ferric iron in disorders of iron overload. we demonstrated that the drug is a potent inhibitor of DNA synthesis by B and T lymphocytes in vitro, likely by impairing the activity of the iron-containing enzyme ribonucleotide reductase [2]. More recently, we have shown that DFO can induce monocyte-macrophage cell differentiation in the human promyelocytic leukemic cell line. HL-60, as judged by altered expression of cell surface antigens, non-specific esterase activity, and morphological changes combined with irreversible inhibition of colony formation [3]. The mechanism appeared to be related to inhibition of replicative DNA synthesis. or due to an increase in number of single-stored DNA breaks. Because DFO has antiproliferative properties as well as modulatory effects on cell differentiation, it is useful for experimental and therapeutic applications.

In vitro, we were able to demonstrate that the anti-proliferative effects of DFO on leukemic cell growth from our patient using a blast colony assay. Because the plating efficiency of PB blast cells was very high, clear-cut suppression of colony growth by DFO could be readily demonstrated. The DFO-induced change in blast cell surface antigen expression from lymphoid to myelomonocytic was striking, and confirmed the modulatory effect of DFO on cell differentiation.

The in vitro changes correlated closely with the findings during the first 48 hours of continuous infusion of DFO when used as a single agent. The PB blast count, that had risen dramatically for 3 days prior to DFO therapy, leveled off after initiation of DFO infusion, suggesting an in vivo antiproliferative effect. Moreover, surface marker antigen changes were observed that were identical to those seen after DFO treatment in vitro. There was convincing evidence for expression of myelomonocytic antigens and concurrent disappearance of lymphoid markers. The possibility that during the 48 hours of DFO treatment a change in PB cell distribution was responsible for the change in surface markers was unlikely since the blast count remained stable during this period. With the HL-60 cell line, Boyd and Metcalf showed that the differentiation process is associated with irreversible loss of proliferative ability, inhibition of DNA synthesis and $G_0/G_1$ cell cycle arrest [9]. Since identical changes in the same cell line were induced by DFO [3], this could be the mechanism by which DFO affected our patient's leukemic cells.

The cellular lineage of the patient's blasts is of interest. The abnormal karyotype that was demonstrated has been observed in ALL and ANLL. It is possible that the blasts showed "lineage infidelity" and expressed markers from two lineages [20, 21]. Another explanation is that the leukemia was myelo-monocytic in origin with "repressed" antigen expression that could only be identified after DFO-induced differentiation. A final possibility, although less likely, is that two separate leukemic populations were responsible for the dual marker findings.

A low plating efficiency of normal pluripotent hematopoietic precursors in acute leukemia, as demonstrated in our patient, has been previously described [22,23]. The significantly higher plating efficiency observed using the CFU-GEMM assay of erythroid as well as granulocyte-macrophage elements from our patient's PB following DFO treatment occurred in spite of persistently high numbers of PB blast cells. It is unlikely that a high percentage of the blast-like cells were actually normal hematopoietic progenitors since their leukemic nature could be demonstrated cytochemically. It is possible that DFO in vivo induced a change in the leukemic blasts allowing their differentiation towards the erythroid as well as the granulocytemacrophage lineage as previously described in Ph[1] positive chronic myelogenous leukemia [24,25]. Another interpretation is that DFO treatment in some manner permitted the proliferation of normal hematopoietic progeniters which were suppressed by the leukemic cells as described by Broxmeyer et al [26,27].

The rationale for using Ara-C with DFO in the treatment protocol was based on the hypothesis that if DFO could block leukemic cells in S-phase, after removal of DFO, Ara-C should destroy the cells progressing through S-phase because its cytotoxic action is S-phase-dependent. From the available data it is still unclear whether the apparent synergism between DFO and Ara-C is attributed to S-phase synchronisation or to the differentiation action of DFO. The marked clinical reduction in PB blast numbers, the changes in leukemic cell marker expression, and the in vitro and in vivo anti-proliferative properties, prompt further experimental and clinical studies with DFO.

## Effects of DFO Alone on the Colony Formatin of Bone Marrow Cells

DFO has a significant inhibitory effect on the colony formation of normal bone marrow cells. The tests were performed according to Z. Estrov et al. (28.).

The results obtained with the CFU-E, BFU-E and CFU-C are shown in Table 6.

## Effect of DFO on Colony Growth of HL-60 Cells

DFO has a significant inhibitory effect on the colony growth of the leukemia cell line HL-60. The test was performed as follows:

HL-60 cells, a promyeloctic leukemia cell line originally developed by Dr. R. Gallo, NIH, were used. Cells were grown in RPMI supplemented with 10 % heat inactivated fetal calf serum (FCS, Flow Laboratories, Rockville, MO) and 2 mM glutamine (Gibco, Berkely, CA), at densities ranging from $1-2 \times 10^5$/ml to $1-10^6$/ml, in a 5 % $CO_2$ humidified atmosphere at 37°C.

HL-60 cells ($10^4$/ml) were plated in 0.8 % methycellulose in RPMI-1640 medium supplemented with 20 % heat inactivated (56°C, 30 min) fetal calf serum at a final volume of 1 ml/culture plate. Cultures were set up in duplicates and maintained at 37°C in a 5 % $CO_2$ humidified atmosphere. Deferoxamine was dissolved in iron-free RPMI culture medium and added at the initiation of culture at different concentrations (1-10 μM). Colonies were counted at 7 days using an inverted microscope. A cluster of more than 20 cells was defined as a colony. Individual colonies were plucked from culture plates spread on glass slides and morphologically assessed after preparation with Wright's stain and non-specific esterase in the presence of fluoride (29). The self renewal capacity of cells was assessed according to McCulloch et al (30). RPMI-1640 medium was added to culture dishes for 2 hrs; the contents of each dish were then transferred into a tube, centrifuged and washed with medium three times. Cells from each dish were counted separately, and viability was assessed by trypan blue dye exclusion. Cells ($1 \times 10^4$/ml) from each dish were recultured in 0.8 % methylcellulose as before.

Ferrioxamine dissolved in RPMI and added to cultures at a concentration of 10 μm did not affect colony formation, proving the iron-dependent mechanism of the DFO effect.

The results obtained are shown in Table 7.

Synergistic Effects of DFO and Other Cytostatic Compounds in vitro

In order to determine the effect of DFO in combination with other cytostatically active compounds the test described before has been performed in the presence of various cytostatic compounds.

Cytosine arabinoside (Cytosar, Upjohn, Toronto, Canada) was dissolved in RPMI and added to cultures in final concentration of $10^{-10}$ to $5 \times 10^{-9}$ M.

Methotrexate (Sigma Chemicals, St. Louis, MO) was dissolved in double distilled water and then in RPMI. Final concentrations of $10^{-8}$ to $10^{-5}$ M were added directly to cultures.

Daunorubicin (Cérubidine SPECIA, Paris, France) was diluted in double distilled water and then in RPMI. Drug concentration ranged from $3.3 \times 10^{-9}$ to $1 \times 10^{-6}$M.

All the drugs tested in our study except daunorubicin were added directly to the cultures in the final concentration as described. Cells ($10^6$ cells/ml) were incubated in various concentrations of daunorubicin at 37°C in 20 % FCS and RPMI for 10 min, then washed x 3 times in cold (4°C) RPMI and added to culture dishes following a trypan-blue dye exlusion viability count.

Cultures were evaluated at day 6 using an inverted microscope (100 x magnification). A cluster of more than 20 cells was determined as a colony.

DFO inhibited HL-60 colony proliferation in a dose-dependent fashion. In the presence of 10 μm DFO a 100 % inhibition was found, 3.5 μM DFO resulted in a 50 % inhibition, while 1 μm did not have any effect on HL-60 colony growth (Table 7).

Cytosine arabinoside inhibited colony growth in a dose-dependent manner. However, when an ineffective dose of DFO (1 μm) was added into cultures containing identical concentrations of the drug, a significantly greater inhibition was demonstrated (Table 10). The DFO enhanced inhibitory effect increased significantly with the increment in cytosine arabinoside concentrations (Table 10, Column 4) proving the synergistic effect of this drug combination.

A similar dose-dependent growth inhibitory effect was found with methotrexate . An increment in the concentrations of methotrexate in the presence of a suboptimal dose of DFO resulted in an enhanced antiproliferative effect causing a significant reduction in colony numbers (Table 11, Column 4). DFO added to cultures of cells which were incubated with daunorubicin for 10 min prior to their plating, induced a similar synergistic enhancement of the antiproliferative effect of this drug (Table 8, Column 4).

The results obtained with 1μM DFO and various concentrations of daunomycin, vincristine, Ara-C and methotrexate are provided in Tables 8, 9, 10 and 11, respectively. With each compound a synergistic effect on the colony growth is observed.

Table 1

The 20-day experimental protocol schedule.

| | Deferoxamine Total Dose | Hours of Infusion | Ara-C Total Dose | WBC x $10^9$/L | Blasts x $10^9$/L |
|---|---|---|---|---|---|
| Day-2 | - | - | - | 16.5 | 3.5 |
| Day-1 | - | - | - | 25.4 | 4.6 |
| Day 0 | - | - | - | 60.9 | 18.2 |
| Day 1 | 720 | 24 | - | 62.5 | 42.2 |
| Day 2 | 720 | 24 | - | | |
| Day 3 | 360 | 12 | 15 | 63.9 | 40.9 |
| Day 4 | 360 | 12 | 15 | | |
| Day 5 | 360 | 12 | 15 | | |
| Day 6 | 360 | 12 | 15 | 47.3 | 40.6 |
| Day 7 | - | - | 15 | | |
| Day 8 | - | - | 15 | 10.9 | 3.1 |
| Day 9 | - | - | 15 | | |
| Day 10 | - | - | 15 | 6.3 | 1.5 |
| Day 11 | - | - | 15 | | |
| Day 12 | - | - | 15 | | |
| Day 13 | - | - | 15 | | |
| Day 14 | 360 | 12 | 15 | | |
| Day 15 | - | - | 15 | 4.1 | 0.2 |
| Day 16 | 360 | 12 | - | 6.5 | 1.4 |
| Day 17 | 1080 | 24 | - | 4.6 | 0.2 |
| Day 18 | 720 | 24 | - | | |
| Day 19 | 720 | 24 | - | | |
| Day 20 | 720 | 24 | - | 4.5 | 0.1 |

Deferoxamine (DFO) was given by continuous infusion 10 mg/kg/hr Cytosine-arabinoside (Ara-C) was given by i.v. push, 5 mg/kg/dose.

Table 2

Surface Markers Analysis of Leukemia Cell Colonies

| | Without Deferoxamine | With Deferoxamine |
|---|---|---|
| Ia | 80 % Positive +++ | 80 % Positive +++ |
| CALLA | 0 % | 0 % |
| B4 | 80 % Positive +++ | 30 % Positive +++ |
| OKM1 | 0 % | > 90 % Positive +++ |
| MY-4 | 0 % | 60 % Positive ++ |
| Leu-1 | 0 % | 0 % |
| Leu-4 | 0 % | 0 % |

PB leukemia cell colonies were plucked at random from cultures containing 2.5 µM deferoxamine compared to cultures without deferoxamine using the immunocytochemical assay.

Table 3

Surface Marker Analysis Pre- and Post-Therapy

| | Before Deferoxamine Treatment | After Deferoxamine Treatment |
|---|---|---|
| Ia | 80 % +++ | 80 % +++ |
| CALLA | 2 % + | 0 % |
| B4 | 37 % ++ | 0 % |
| OKM1 | 0 % | 40 % +++ |
| MY-4 | 8 % + | 60 % +++ |
| Leu-4 | 0 % | 0 % |
| Leu-1 | 0 % | 0 % |

Immunologic surface markers were dermined before and after 48 hours of deferoxamine treatment on PB smears using the immunocytochemical method

Table 4

Surface Marker Analysis Before and at Conclusion of 20 day Treatment

% Positive Cells

| | Before Deferoxamine Treatment | After Deferoxamine Treatment |
|---|---|---|
| Ia | 80 % | 75 % |
| sIg | 10 % | 0 % |
| BA-1 | 14 % | 0 % |
| BA-2 | 50 % | 0 % |
| B1 | Negative | Negative |
| B4 | 68 % | 10 % |
| CALLA | Negative | Negative |
| E-Rosettes | Negative | Negative |
| Leu-1 | Negative | Negative |
| MY-7 | 0 % | 20 % |
| Mol | 0 % | 20 % |
| MY-4 | 10 % | 20 % |

Immunologic surface markers were determined by immunofluorescence before and at the conclusion of the 20-day treatment course on bone marrow cells.

## Table 5

### Numbers of CFU-GEMM Colonies Pre- and Post-Therapy

| Colony Type | Before Deferoxamine Treatment | After Deferoxamine Treatment |
|---|---|---|
| BFU-E | 9 ± 1 | 113 ± 14 |
| CFU-C | 45 ± 6 | 126 ± 17 |
| Mixed | 1 ± 1 | 5 ± 2 |

CFU-GEMM colony culture assay of patients PB were performed before and after 48 hours deferoxamine treatment.

## Table 6

### Effect of Deferoxamine on Bone Marrow Colony Formation

Colony Number
Deferoxamine Concentration (µM)

| | 0 | 5 | 10 | 15 | 20 |
|---|---|---|---|---|---|
| BFU-E | 38 | 21 | 12 | 4 | 0 |
| CFU-E | 170 | 95 | 70 | 50 | 17 |
| CFU-C | 48 | 30 | 10 | 2 | 0 |

## Table 7

### Effect of Deferoxamine on HL-60 Colony Growth

| Deferoxamine conc. (µM) | Colony Number |
|---|---|
| 0 | 960 |
| 1.0 | 980 |
| 3.3 | 461 |
| 5.0 | 317 |
| 7.3 | 72 |
| 10.0 | 0 |

Table 8

Synergistic Effects of DFO and Daunorubicin on HL-60 Colony Growth

### Colony Growth, DFO (1μM)

| conc. Daunomycin (M) | - | + | % Enhanced Inhibition |
|---|---|---|---|
| 0 | 1080 ± 150 | 1020 ± 250 | 6 |
| $3.3 \times 10^{-9}$ | 800 ± 50 | 720 ± 180 | 9 |
| $1 \times 10^{-8}$ | 610 ± 50 | 520 ± 40 | 15 |
| $3.3 \times 10^{-8}$ | 570 ± 50 | 370 ± 40 | 35 |
| $6.6 \times 10^{-8}$ | 400 ± 50 | 240 ± 20 | 40 |
| $1 \times 10^{-7}$ | 340 ± 80 | 170 ± 40 | 50 |
| $1 \times 10^{-6}$ | 50 ± 30 | 0 | 100 |

Table 9

Synergistic Effects of DFO and Vincristine on HL-60 Colony Growth

### Colony Number, DFO (1μM)

| conc. Vincristine (M) | - | + | % Enhanced Inhibition |
|---|---|---|---|
| 0 | 510 ± 10 | 500 ± 30 | 2 |
| $10^{-5}$ | 520 ± 10 | 470 ± 20 | 10 |
| $10^{-4}$ | 310 ± 20 | 110 ± 10 | 64 |
| $10^{-3}$ | 3 ± 3 | 0 | -- |
| $10^{-2}$ | 0 | 0 | -- |

Table 10

Synergistic Effects of DFO and Ara-C on HL-60 Colony Growth

| conc. Ara-C (M) | Colony Number, DFO (1μM) | | % Enhanced Inhibition |
| | − | + | |
| --- | --- | --- | --- |
| 0 | 2700 ± 165 | 2540 ± 60 | 6 |
| $1 \times 10^{-10}$ | 2630 ± 115 | 2285 ± 120 | 13 |
| $5 \times 10^{-10}$ | 2775 ± 30 | 2100 ± 110 | 24 |
| $1 \times 10^{-9}$ | 2370 ± 105 | 1275 ± 100 | 46 |
| $2.5 \times 10^{-9}$ | 290 ± 55 | 80 ± 15 | 73 |
| $5 \times 10^{-9}$ | 2 ± 1 | 0 | − |

Table 11

Synergistic Effects of DFO and Methotrexate on HL-60 Colony Growth

| conc. Methotrexate M | Colony Number, DFO (1μM) | | % Enhanced Inhibition |
| | − | + | |
| --- | --- | --- | --- |
| 0 | 1825 ± 55 | 1845 ± 145 | − |
| $1 \times 10^{-8}$ | 1420 ± 95 | 1310 ± 70 | 8 |
| $1 \times 10^{-7}$ | 1245 ± 35 | 1115 ± 80 | 10 |
| $5 \times 10^{-7}$ | 1100 ± 70 | 765 ± 55 | 30 |
| $1 \times 10^{-6}$ | 1015 ± 90 | 730 ± 90 | 28 |
| $3.3 \times 10^{-6}$ | 750 ± 60 | 400 ± 55 | 47 |
| $1 \times 10^{-5}$ | 670 ± 55 | 290 ± 90 | 57 |

Synergistic Effect of DFP and Ara-C on Tumour Growth in Mice

Female B6D2F1 mice (four groups of 10 mice per group) were injected i.p. with a suspension of L-1210 tumour cells on day 0. Physicological saline, DFO (in distilled water) and Ara-C (in distilled water) were administered either by continuous infusion using a minipump Osmet 2001 or at once according to the following schedule:

group 1: control group; starting day 2; physiological saline, a total of 200 μl with a release rate of 1 μl/hour, s.c. by a minipump

group 2: on day 2, 400 mg/kg/day DFO, s.c. by a minipump

group 3: on day 6, 200 mg/kg Ara-C, i.p. at once

group 4: on day 2, 400 mg/kg/day DFO. s.c. by a minipump, followed on day 6 by 200 mg/kg Ara-C, i.p. at once.

One hour prior to kill on day 8 the mice received one ip injection of 0.002 mCi of 3H-thymidine per g body weight. Cells were harvested from the peritoneal cavity, the volume of ascites measured and the cell counts determined in a coulter counter, after lysis of red cells with zap-oglobin, for calculation of total tumour cell numbers. Also, of each sample cell smears were done, Feulgen stained and autoradiographed, whereby the number and percentage of labelled cells was determined and used as a measure for proliferating cells. The results are compiled in Table 12.

## Table 12

### Synergistic Effect of DFO and Ara-C on Tumour Growth in Mice

| Treatment group | Number of cells in the peritoneal cavity | | Number of proliferating cells in peritoneal cavity | |
|---|---|---|---|---|
| | $\bar{x} \pm$ SD $(10^8)$ | % of control | $\bar{x} \pm$ SD $(10^8)$ | % of control |
| Group 1: control saline in minipump | 3.68 ± 1.37 | 100 | 1.22 ± 0.64 | 100 |
| Group 2: DFO 400 mg/kg/d in minipump | 3.49 ± 1.75 | 95 | 0.98 ± 0.64 | 80 |
| Group 3: Ara-C 200 mg/kg once ip | 1.11 ± 0.40 * | 30 | 0.29 ± 0.10 * | 24 |
| Group 4: DFO 400 mg/kg/d in minipump + Ara-C 200 mg/kg ip | 0.54 ± 0.17 *+ | 15 | 0.11 ± 0.04 *+ | 9 |

\* significantly different from control group, p <0.005

+ significantly different from group 3 (only Ara-C) as well as from group 2 (only DFO) p <0.005

Conclusion

DFO alone in the given dosage constantly infused for 6 days hardly affects the total tumour cell number, though the proportions of proliferating cells was decreased to 28 % ± 8 %, indicative of DFO affecting S-phase.

Ara-C alone reduces the total cell number to 30 % with 26 % ± 6 % proportions of proliferating cells two days after a single administration.

The consecutive administration of DFO and Ara-C reduced the total tumour cell number to 15 % and so is the number of proliferating cells, the proportion of which was 22 % ± 6 %.

The experiment shows that DFO was a potentiating effect on Ara-C, though it was not effective by itself in the administered dosage.

Synergistic Effect of DFO and 10-EDAM on Tumour Growth and Metastases of Lewis Lung Carcinoma in Mice

Female C57B16 mice (10 mice/group, 18-22 g body weight) were i.m. injected into the left hind leg with 0.1 ml of a tumour cell suspension of Lewis lung carcinoma (day 0).

Nine groups of animals were investigated. The test compounds were s.c. administered as follows: physiological saline: once daily for eleven days starting from day 0 up to day 10.

DFO: 0.5 ml in distilled water in a dose of 10 and 30 mg/kg once daily for eleven days, on day 0 up to day 10 after tumour inocculation.

10-EDAM: 0.5 ml in distilled water in a dose of 3 and 6 mg/kg, once daily on days 1, 3, 5, 7 and 9 after tumour inocculation.

Both hind legs were removed from the body. weighed, and the difference between the tumour bearing and the tumour free leg was taken as the tumour weight of one ididvidual mouse.

Lungs were filled up with Pelikan-ink via the trachea, fixed in Fekete-solution and metastases (white spot on black back-ground) on the lung surface were counted microscopically.

The results are compiled in Table 13.

**Table 13**

Synergistic Effect of DFO and 10-EDAM on Tumour Growth and Metastases of Lewis Lung Carcinoma

| Treatment group | Compound | Dose (mg/kg) | Number of injections | Tumour weight | | Number of lung metastases | |
|---|---|---|---|---|---|---|---|
| | | | | $\bar{x} \pm$ SD (g) | % of control | $\bar{x} \pm$ SD | % of control |
| 1 | saline | 0 | 11 | 7.52 ± 0.69 | 100 | 67 ± 25 | 100 |
| 2 | DFO | 10 | 11 | 7.56 ± 0.32 | 100 | 69 ± 26 | 103 |
| 3 | DFO | 30 | 11 | 7.99 ± 0.42 | 106 | 52 ± 16 | 78 |
| 4 | 10-EDAM | 3 | 5 | 6.87 ± 0.47 | 91 | 44 ± 18 | 66 |
| 5 | 10-EDAM | 6 | 5 | 6.70 ± 1.06 | 89 | 23 ± 10 | 34 |
| 6 | DFO 10-EDAM | 10 3 | 11 5 | 7.05 ± 0.56 | 93 | 20 ± 8 | 30 |
| 7 | DFO 10-EDAM | 10 6 | 11 5 | 5.65 ± 1.26 | 75 | 7 ± 4 | 10 |
| 8 | DFO 10-EDAM | 30 3 | 11 5 | 6.46 ± 0.80 | 85 | 25 ± 15 | 37 |
| 9 | DFO 10-EDAM | 30 6 | 11 5 | 6.26 ± 0.75 | 83 | 7 ± 2 | 10 |

0 227 593

## Conclusion

DFO alone in the adminstered dose had no effect on the weight of the primary tumour and the number of metastases (group 2 and 3).

10-EDAM alone in the administered dose had no effect on the weight of the primary tumour and only marginal effects on the number of metastases (groups 4 and 5).

DFO and 10-EDAM in combination of dosages where the single compounds are ineffective have a marginal effect on the weight of the tumour and a significant effect on the number of metastases (groups 6 to 9).

The experiment inidcates a synergistic antitumour effect of DFO and 10-EDAM combinations.

## Pharmaceutical Preparations

Based on the description hereinbefore, therapeutic combinations and the preparation thereof are also within the ambit of the present invention. Thus, the invention concerns a therapeutic combination consisting essentially of a therapeutically effective amount of an iron chelator and a therapeutically effective amount or a lower than therapeutically effective amount of a cytostatic agent, which components are present either separately in form of their pharmaceutical preparations or together in a combined pharmaceutical preparation, which preparations may contain at least one pharmaceutically acceptable carrier. The therapeutic combinations may consist of two individual components of which each contains the iron chelator and the cytostatic agent, respectively, in form of a pharmaceutical preparation conventional for each of the agents, whereby the cytostatic agent may be present in a lower than conventional amount. Alternatively it is also possible to combine the iron chelator and the cytostatic agent in one pharmaceutical preparation which is, however, only feasible in the case that both active agents are applicable by the same route. If the iron chelator is applied parenterally it can only be admixed with those cytostatic agents which likewise are to be used parenterally. The two component therapeutic combination is of course necessary if the administrative modes or routes differ, e.g. if the cytostatic agent is applied orally and the iron chelator parenterally, or if the cytostatic agent is administered by bolus injection and the iron chelator is infused. In the case of two components the therapeutic combination may be administered in a prescribed timely interval. For example it may be of advantage to administer first the iron chelator by infusion during one or several days in a normally applied dosage, whereupon the cytostatic is administered either parenterally or orally in a normal or lower than normal dosage regimen.

In general the pharmaceutical preparation contains effective amounts of the two active ingredients together or in admixture with inorganic or organic, solid or liquid, pharmaceutically acceptable carriers which are suitable preferably for parenteral administration.

The active compounds of the present invention are preferably used in the form of preparations or infusion solutions for parenteral, for example intramuscular or intravenous, administration. Such solutions are preferably isotonic aqueous solutions or suspensions which can be prepared before use, for example from lyophilised preparations which contain the active ingredient alone or together with a pharmaceutically acceptable carrier. The pharmaceutical preparations may be sterilised and/or contain adjuncts, for example preservatives, stabilisers, wetting agents and/or emlusifiers, solubilisers, salts for regulating the osmotic pressure and/or buffers. The present pharmaceutical preparations, which may, if desired, contain further pharmacologically valuable substances, are produced in a manner known per se, for example by means of conventional dissolving or lyophilising processes, and contain from approximately 0.1 % to 100 %, especially from approximately 1 % to approximately 50 %, and in the case of lyophilisates up to 100 %, of the active ingredient.

In particular the invention concerns a therapeutic combination, wherein the iron chelator is deferoxamine methansulfonate, the dosage unit form of which consists of ampoules containing 0,5 g or 3 g active compound, and the cytostatic agent is cytarabine (cytosine-arabinoside) the dosage unit form of which consists of ampoules containing 100 mg or 500 mg active compound.

The invention concerns further the use of the therapeutic combinations for the treatment of cancer, especially leukemia.

The invention concerns also the use of an iron chelator, especially deferoxamine or a salt thereof, for example the methanesulphonate, for the treatment of cancer, especially leukemia and for the manufacture of a medicament for the treatment of cancer, especially leukemia, which latter may contain instructions for its use, and wherein the cytostatic may be used in a lower than normally applied dose.

The inventions concerns also the use of an iron chelator, especially deferoxamine or a salt thereof, and a cytostatic agent for the manufacture of a medicament for the treatment of cancer, especially leukemia, and/or for the prevention or alleviation of adverse effects due to the cytostatic agent or for the prophylactic treatment of cancer, especially leukemia, and wherein the iron chelator and the cytostatic agent maybe two separate components, and which may inlcude instructions for use which stipulate that the iron chelator should be administered first.

The invention concerns also a preparation or pack comprising
(i) an iron chelator, especially deferoxamine or a salt thereof, for example the methanesulphonate, and
(ii) a cytostatic agent,
in combination or separate for the use together or sequentially in the treatment of cancer, especially leukemia, and/or for the prevention or alleviation of adverse effects due to the cytostatic agent, wherein the routes of administration of the two ingredients may be different, and which may include instructions for use which

17

0 227 593

stipulate that the iron chelator should be used first.

Following is an example of a therapeutic combination according to the invention which, however, should not be construed as a limitation thereof.

Example 1: Therapeutic combination for separate administration

Component A: Dry ampoules containing 0.5 g or 3 g iron chelator Ampoules of 5 ml or 50 ml, respectively, volume are filled with 5 ml or 30 ml, respectively. of sterile filtered 10 % (w/v) aqueous solution of deferoxamine methanesulfonate and lyophilized. The infusion solution is prepared by adding the respective volume (5 or 30 ml) of sterile water or physiological saline.

Component B: Dry ampoules containing 100 mg or 500 mg of cytarabin Ampoules of 10 ml or 20 ml. respectively, volume are filled with 2 ml or 10 ml, respectively. of a 5 % (w/v) aqueous solution of cytarabin and lyophilized. The injection solution is prepared by adding 5 ml or 10 ml, respectively; of sterile water or physiological saline.

The therapeutic combination contains the desired number of ampoules necessary for one course of treatment, e.g. for 14 days, and optional instructions for application which stipulate that the iron chelator should be used first.

References

1. Modell B, Letsky EA, Flynn DM, Peto R, Weatherall DJ: Survival and desferrioxamine in thalassaemia major. Br.Med.J.284:1081, 1982.

2. Lederman HM, Cohen A, Lee JWW, Freedman MH, Gelfand EW: Deferoxamine: reversible S-phase inhibitor of human lymphocyte proliferation. Blood 64:748-753, 1984.

3. Kaplinsky C, Estrov Z, Freedman MH, Gelfand EW, Cohen A: Effect of deferoxamine on DNA synthesis. DNA repair, cell proliferation and differentiation of HL-60 cells. Blood, ...... 1985.

4. Ha K, Hozum N, Hrinen A, Gelfand GW, Linerege specific classification of leukemia: results of the analysis of sixty cases of childhood leukemia, Br. J. Haematol. 1985, 61, 237-249.

5. Fauser AA, Messner HA: Granuloerythropoietic colonies in human bone marrow, peripheral blood, and cord blood. Blood 52:1243, 1978.

6. Messner HA, Fauser AA: culture studies of human pluripotent hemopoietic progenitors. Blut 41:327, 1980.

7. Aye M.T., Niho Y., Till J.E., McCulloch E.A.: Studies of leukemic cell populations in culture. Blood 44:205, 1974.

8. Izaguirre CA, Curtis J, Messner H, McCulloch EA: A colony assay for blast cell progenitors in non-B non-T (common) acute lymphoblastic leukemia. Blood 57:823, 1981.

9. Touw I, Delwel R, Bolhius R, van Zamen G, Löwenberg B: Common and pre-B acute lymphoblastic leukemia cells express interleukin 2 receptors, and interleukin 2 stimulates in vitro colony formation. Blood 66:556-561, 1985.

10. Boyum A: Separation of leukocytes from blood and bone marrow. Scand J Clin Lab Invest 21:7 (suppl), 1968.

11. Shaw GM, Levy PC, LoBuglio AF: Human monocyte antibody-dependent cell mediated cytotoxicity to tumor cells. J Clin Invest 62:1172, 1978.

12. Tucker SB, Pierre RV, Jordan RE: Rapid identification monocytes in mixed mononuclear cell preparation. J Immunol Method 14:267, 1977.

13. Li CY, Ziesmer SC, Yam LT, English MX, Janckila AJ: Practical immunochemical identification of human blood cells. Am J Clin Pathol 81:204, 1984.

14. Yam LT, English MC, Janckila AJ, Ziesmer S, Li CY: Immunocytochemical characterization of human blood cells. Am J Clin Pathol 80:314-321, 1983.

15. Erber WN, Pinching AJ. Mason DY: Immunocytochemical detection of T and B cell populations in routine blood smears. Lancet 1:1042-1046, 1984.

16. Van der Berghe H, David G, Broeckaert-Van Orshover A, Louwagie A, Verwilghen R, Casteels-Van Daele M, Eggermont E, Feckels R: A new chromosome anomaly in acute lymphoblastic leukemia (ALL). Hum Genet 46:173, 1979.

17. Nagasaka M, Maeda S, Maeda H, Chen HL, Kita K, Mabuchi O, Misu H, Matsuo T, Sugiyama T: Four cases of t (4;11) acute leukemia and its myelomonocytic nature in infants. Blood 61:1174-1181, 1983.

18. Arthur DC, Bloomfield CD, Lindquist LL, Nesbit ME: Translocation 4;11 in acute lymphoblastic leukemia: Clinical characteristics and prognostic significance. Blood 59:96, 1982.

19. Boyd AW, Metcalf D: Induction of differentiation in HL-60 leukemic cells: A cell cycle-dependent all-or-none event. Leukemia Res 8:27, 1984.

20. Smith LJ, Curtis JE, Messner HA, Senn JS. Furthrmayr H, McCulloch EA: Lineage infidelity in acute leukemia. Blood 61:1138-1145, 1983.

21. Ha K, Minden M, Hozumi N, Gelfand EW: Immunoglobulin gene rearrangement in acute myelogenous leukemia. Cancer Res 44:4658, 1984.

22. Messner HA: Human stem cells in culture. Clin Haematol 13:393-404, 1984.

23. Minden MD, Golde DW. Takaku F: Hematopoietic stem cells. Hematology, series editor Brinkhous KM. Marcel Dekker, Inc., New York and Basel pp 273-289. 1985.

24. Altman AJ, Baehner RL: In vitro colony-forming characteristics of chronic granulocytic leukemia in childhood. J Pediatr 86:221. 1975.

25. Goto T, Nishikori M, Arlin Z, Gee T, Kempin S, Burchenal J, Strife A, Wisniewski D, Lambek C, Jhanwar S, Chaganti R, Clarkson B: Growth characteristics of leukemic and normal hematopoietic cells in Ph' + chronic myelongenous leukemia and effects of intensive treatment. Blood 59:793, 1982.

26. Broxmeyer HA, Bognacki J, Dorner MH, Sousa M: Identification of leukemia-associated inhibitory activity as acidic isoferritins. A granulocytes and macrophages. J Exp Med 153:1426-1444, 1981.

27. Broxmeyer HA: Relationship of cell cycle expression of Ia like antigenic determinants on normal and leukemia human granulocyte-macrophage progenitor cells to regulation in vitro by acidic isoferritins. J Clin Invest 69:632-642. 1982.

28. Estrov Z, Grunberger T, Chan H, Freedman M, Juvenile Chronic, Myelogenous Leukemia: Characterization of the Disease Using Cell Cultures, Blood, 67:1382-1387, 1986.

29. Yam LT, Li CY. Crosby WH: Cytochemical identification of monocytes and granulocytes. Am. J. Clin. Pathol. 55,283, 1971.

30. McCulloch EA, Curtis JE, Messner HA, Senn JS, Germanson TP: The contribution of blast cell properties to outcome variations in acute myeloblastic leukemia (AML). Blood 49,601, 1982.

## Claims

1. A therapeutical method for the treatment of cancer, characterized in that an iron chelator and a cytostatic agent in a cytostatically effective amount are consecutively or simultaneously administered to a patient suffering from cancer.

2. A therapeutical method according to claim 1, characterized in that the cancer is leukemia.

3. A therapeutical method according to claim 1, characterized in that the iron chelator is deferoxamine, a derivative thereof or a pharmaceutically acceptable salt thereof.

4. A therapeutical method according to claim 1, characterized in that the iron chelator is the methanesulfonate of deferoxamine.

5. A therapeutical method according to claim 1, characterized in that the cytostatic agent is selected from an alkylating cytostatic, such as mechlorethamine, triethylenephosphoramide, cyclophosphamide, ifosfamide, chlorambucil, busulfam, melphalan, or triaziquone, or from a cytostatic nitrosourea, such as carmustine, lomustine or semustine, from an antimetabolite, such as methotrexate, 10-Ethyl-10-deaza-aminopterin, mercaptopurine, cytarabine (cytosinearabinoside), fluorouracil, floxuridine or ftorafur, from cytostatic natural products, such as vinblastine, vincristine, daunorubicin, doxorubicin, methramycin, streptonigrin, mitomycin or bleomycin, from cytostatic other agents, such as cisplatin, procarbazine, hydroxyurea, L-asparaginase, dacarbazine, mitotane, estramustine or podophyllotoxin, or cytostatic hormones, or hormone antagonists, such as corticosteroids, e.g. prednisone, progestins, e.g. hydroxyprogesteron or medroprogesterone, estrogens, e.g. diethylstilbestrol, antiestrogens, e.g. tamoxifen, and androgens, e.g. testosterone, and aromatase inhibitors, e.g. aminogluthetimide.

6. A therapeutical method according to claim 1, characterized in that the cytostatic agent is cytarabine (cytosine arabinoside).

7. A therapeutical method according to claim 1, characterized in that the cytostatic agent is daunorubicin.

8. A therapeutical method according to claim 1, characterized in that the cytostatic agent is vincristine.

9. A therapeutical method according to claim 1, characterized in that the cytostatic agent is methotrexate.

10. A therapeutical method according to claim 1, characterized in that the cytostatic agent is 10-ethyl-10-deaza-aminopterin.

11. A therapeutical method according to claim 1, characterized in that the iron chelator is administered before the cytostatic agent.

12. A therapeutical method according to claim 1, characterized in that the ironchelator is administered during the first one to six or seven days and the cytostatic agent is administered starting at the third day.

13. A therapeutic method according to claim 1, characterized in that the iron chelator is administered in dosages normally given to bind the freely available iron.

14. A therapeutic method according to claim 1, characterized in that the cytostatic agent is administered in dosages lower than normally applied.

15. A therapeutic combination consisting essentially of a therapeutically effective amount of an iron chelator and a therapeutically effective amount or a lower than therapeutically effective amount of a cytostatic agent. which components are present either separately in form of their pharmaceutical preparations or together in a combined pharmaceutical preparation, which preparations may contain at least one pharmaceutically acceptable carrier.

16. A therapeutic combination according to claim 15, wherein the iron chelator and the cytostatic agent is selected from the compounds mentioned in any one of claims 3 to 10.

17. A therapeutic combination according to claim 15, wherein the iron chelator is present in dosage unit forms normally used.

18. A therapeutic combination according to claim 15. wherein the cytostatic agent is present in dosage unit forms normally used.

19. A therapeutic combination according to claim 15, wherein the cytostatic agent is present in dosage unit forms containing a lower amount than normally used.

20. A therapeutic combination according to claim 15. wherein the iron chelator is deferoxamine methansulfonate. the dosage unit form of which consists of ampoules containing 0,5 g or 3 g active comopund. and the cytostatic agent is cytarabine (cytosine-arabinoside) the dosage unit form of which consists of ampoules containing 100 mg or 500 mg active compound.

21. Use of the therapeutic combinations according to any one of claims 15 to 20 for the treatment of cancer.

22. Use of the therapeutic combinations according to any one of claims 15 to 20 for the treatment of leukemia.

23. Process for the preparation of therapeutic combinations according to any one fo claims 15 to 20, characterized in that an iron chelator and a cytostatic agent mentioned in any one of claims 1 or 3 to 10 are pharmaceutically processed according to conventional methods.

24. Use of an iron chelator, especially deferoxamine or a salt thereof, for example the methanesulphonate, for the treatment of cancer, especially leukemia.

25. The use of an iron chelator, especially deferoxamine or a salt thereof, for example the methanesulphonate, for the manufacture of a medicament for the treatment of cancer, especially leukemia.

26. The use of an iron chelator, especially deferoxamine or a salt thereof, for example the methanesulphonate, for the manufacture of a medicament for the treatment of cancer, especially leukemia, and instructions for its use.

27. A use as claimed in any one fo claims 24 to 26, wherein the iron chelator is used in conjunction with a cytostatic agent, especially in a lower than normally applied dose of the cytostatic agent.

28. The use of an iron chelator, especially deferoxamine or a salt thereof, and a cytostatic agent for the manufacture of a medicament for the treatment of cancer, especially leukemia, and/or for the prevention or alleviation of adverse effects due to the cytostatic agent.

29. The use of an iron chelator, especially deferoxamine or a salt thereof, and optionally a cytostatic agent, for the manufacture of a medicament for the therapeutic and/or prophylactic treatment of cancer, especially leukemia.

30. A use as claimed in any one of claims 27 to 29, wherein the iron chelator and the cytostatic agent are two separate components.

31. A use as claimed in claim 30 which includes instructions for use which stipulate that the iron chelator should be administered first.

32. A preparation or pack comprising

(i) an iron chelator, especially deferoxamine or a salt thereof, for example the methanesulphonate. and

(ii) a cytostatic agent,

in combination or separate for the use together or sequentially in the treatment of cancer, especially leukemia, and/or for the prevention of alleviation of adverse effects due to the cytostatic agent.

33. A pack as claimed in claim 32. wherein the routes of administration of the two ingredients are different.

34. A pack as claimed in claim 32 or claim 33, which includes instructions for use which stipulate that the iron chelator should be used first.

European Patent Office

# PARTIAL EUROPEAN SEARCH REPORT

which under Rule 45 of the European Patent Convention shall be considered, for the purposes of subsequent proceedings, as the European search report

Application number

EP 86 81 0529

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | CHEMICAL ABSTRACTS, vol. 101, no. 11, September 10, 1984, page 32, ref.no. 83686f; Columbus, Ohio, US A. SATO et al.: "Synergistic inhibition of leukemia L1210 cell growth in vitro by combinations of 2-fluoroadenine nucleosides and hydroxyurea or 2,3-dihydro-1H-pyrazole(2,3-a)imidazole." & CANCER RES. 1984, 44(8), 3286-90. <br><br> * Abstract * <br><br> --- | 15-20, 23,27-34 | A 61 K 45/06 <br><br> A 61 K 31/16 <br><br> A 61 K 31/70// <br><br> (A 61 K 31/70 <br>    31:16) |
| X | CHEMICAL ABSTRACTS, vol. 99, no. 13, September 26, 1983, page 31, ref.no. 98888n; Columbus, Ohio, US A. SATO et al.: "Effects of biochemical modulation of drug combinations directed at the ribonucleotide reductase site on leukemia L1210 cell growth in culture." & ADV. ENZYME REGUL. 1983, 21, 259-70. <br><br> * Abstract * | 15-20, 23,27-34 | **TECHNICAL FIELDS SEARCHED (Int. Cl.4)** <br><br> A 61 K |

## INCOMPLETE SEARCH    ---    ./.

The Search Division considers that the present European patent application does not comply with the provisions of the European Patent Convention to such an extent that it is not possible to carry out a meaningful search into the state of the art on the basis of some of the claims.

Claims searched completely:    15-20,23,25-34

Claims searched incompletely:

Claims not searched:    1-14,21,22,24

Reason for the limitation of the search:

Method for treatment of the human or animal body by surgery or therapy (see art. 52(4) of the European Patent Convention).

| Place of search | Date of completion of the search | Examiner |
|---|---|---|
| The Hague | 27-02-1987 | BRINKMANN |

EPO Form 1505.1 03.82

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

| Category | Citation of document with indication, where appropriate, of relevant passages | Relevant to claim |
|---|---|---|
| X | CHEMICAL ABSTRACTS, vol. 98, no. 25, June 20, 1983, page 375, ref.no. 213422z; Columbus, Ohio, US R. GAMBARI et al.: "Desferrioxamine inhibits induced erythroid differentiation of human leukemic K-562 cells." & CELL DIFFER. 1983, 12(5), 249-55. * Abstract * | 25,26 |
| | --- | |
| X | CHEMICAL ABSTRACTS, vol. 104, no. 7, February 17, 1986, page 410, ref.no. 49627q; Columbus, Ohio, US Y. CHINEN et al.: "Increased complement sensitivity of cell line K562 induced by deferoxamine." & IGAKU NO AYUMI 1985, 135(1), 65-6. * Abstract * | 25,26 |
| | --- | |
| X | EP-A-0 068 314 (CIBA-GEIGY AG) * Whole document * | 15-20, 23,25-34 |
| | ------------------- | |

**DOCUMENTS CONSIDERED TO BE RELEVANT**

**CLASSIFICATION OF THE APPLICATION (Int. Cl.4)**

**TECHNICAL FIELDS SEARCHED (Int. Cl 4)**

EPO Form 1505.3  06.78